# FASCICULE DE BREVET EUROPEEN

(11) **EP 2 964 076 B1**
(45) Date de publication et mention de la délivrance du brevet: **04.04.2018**
(21) Numéro de dépôt: 14713201.3
(22) Date de dépôt: 28.02.2014
(51) Int. Cl.: A61B 5/00

(54) **DISPOSITIF D'OBTURATION D'UN CONTENANT COMPRENANT UNE PIECE D'ADAPTATION POUR RECEVOIR UN DISPOSITIF ELECTROPORTATIF**
VORRICHTUNG ZUM ABDICHTEN EINES BEHÄLTERS MIT EINEM ADAPTERTEIL ZUR AUFNAHME EINER TRAGBAREN ELEKTRISCHEN VORRICHTUNG
DEVICE FOR SEALING A CONTAINER COMPRISING AN ADAPTER PART FOR RECEIVING A PORTABLE ELECTRIC DEVICE

(30) Priorité: 08.03.2013 FR 1352123
(43) Date de publication de la demande: 13.01.2016
(73) Titulaire: Intuiskin, 38926 Crolles (FR)
(72) Inventeur: KARAM, Jean-Michel, F-38330 Biviers (FR); RÄNSCH, Pascal, F-38240 Meylan (FR); MOULINIER, David, F-69100 Villeurbanne (FR)
(74) Mandataire: Cabinet Laurent & Charras
(86) Numéro de dépôt international: PCT/FR2014/050448
(87) Numéro de publication internationale: WO 2014/135774

(56) Documents cités:
- WO-A1-2013/028731
- FR-A1- 2 895 377
- US-A1- 2010 302 358

## Description

### Domaine technique

L'invention se rapporte au domaine de la cosmétologie et de la dermatologie. Elle vise plus particulièrement un dispositif d'obturation d'un contenant tel un flacon, tube ou analogue contenant un produit cosmétique, dermatologique, colorant pour cheveux ou encore du maquillage. Un tel dispositif d'obturation permet en outre de faciliter la réalisation d'un cliché photographique d'une surface corporelle d'un utilisateur. Une telle surface corporelle peut être une surface cutanée ou encore une surface capillaire.

Un tel dispositif d'obturation peut notamment comporter une pièce d'adaptation permettant le positionnement précis d'un dispositif électroportatif indépendant par rapport au corps du dispositif d'obturation, le dispositif électroportatif étant apte à prendre une image de la surface corporelle.

### Techniques antérieures

De façon générale, les appareils permettant de réaliser une image d'une surface corporelle comportent à la fois une unité de traitement informatisée et un appareil photographique ou une caméra positionnés dans une sonde qui vient au contact de la surface corporelle.

Un tel appareil a notamment été décrit par le Demandeur dans le document WO 03/037184. Cet appareil est conçu pour être installé à l'intérieur d'un centre dermatologique ou tout autre cabinet d'analyses dermatologiques. Il se présente sous la forme d'un ordinateur équipé de cartes d'acquisition et d'une sonde. Il est donc relativement volumineux et donc difficilement transportable. Par conséquent, le nombre d'utilisateurs potentiels de ce type d'appareil est limité, ou il oblige les personnes intéressées à se rendre dans un lieu équipé d'un tel appareil.

On connaît également, tel que décrit par le Demandeur dans le document WO 2006027530A1, un dispositif d'analyse qui se présente sous la forme d'une carte à puce permettant de fournir une information à l'utilisateur sur un paramètre physico-chimique de la surface cutanée. Les informations délivrées par le capteur sont mémorisées dans la carte. Une telle carte permet d'analyser et de visualiser une information fonction d'au moins un paramètre physico-chimique d'une surface cutanée. Cette mesure peut être réalisée quel que soit l'endroit où l'on se trouve et ce à n'importe quel instant au moyen d'un appareil miniaturisé et autonome.

Cependant une telle carte à puce ne permet pas d'éclairer ou encore de réaliser une image d'une surface corporelle.

On connait également, tel que décrit par le Demandeur dans le document WO 2007/074305, un flacon et un bouchon équipé d'une caméra et d'une source lumineuse permettant l'évaluation des caractéristiques dimensionnelles ou colorimétriques de la peau. Cependant, une telle camera ne permet pas de réaliser une image grossie de la peau et/ou est très couteuse à produire, ce qui est alors incompatible avec un dispositif d'obturation à vocation jetable.

De plus, même si un objectif à mise au point rapprochée du type macro peut être utilisé en combinaison avec la caméra, rien n'indique que cet objectif permet de définir un volume sensiblement étanche au-dessus de l'orifice du contenant.

Le but de l'invention est donc de fournir à un utilisateur final un outil quasi-autonome miniaturisé permettant de prendre une image grossie d'une surface corporelle à l'aide d'un dispositif électroportatif annexe. Un tel outil est ainsi constitué par un dispositif d'obturation, avantageusement jetable, d'un contenant pour une substance à appliquer au niveau de la surface corporelle.

### Exposé de l'invention

L'invention concerne donc un dispositif d'obturation d'un contenant, le dispositif comportant un corps apte à recouvrir et protéger un orifice du contenant. Le corps présente une surface plane destinée à venir au contact d'une surface plane parallèle du contenant.

Selon l'invention, le dispositif d'obturation se caractérise en ce qu'il comporte :
- un système optique permettant à la fois de définir un volume sensiblement étanche au-dessus de l'orifice du contenant et de réaliser une image grossie d'une surface corporelle d'un utilisateur du dispositif, lorsque la surface corporelle est positionnée au contact de la surface plane du corps ;
- un logement apte à recevoir un dispositif électroportatif comportant :
   - un capteur optique permettant de prendre ladite image de ladite surface corporelle, ledit logement étant agencé de telle sorte que le capteur optique du dispositif électroportatif est en regard du système optique ;
   - une unité de stockage permettant de mémoriser ladite image, et ;
   - une unité de transfert des données de ladite image.

Autrement dit, la distance focale du système optique permet de réaliser un grossissement de la surface corporelle. Un tel système optique peut consister notamment en doublet de lentilles agencées parallèlement de façon coaxiales.

Le volume étanche, notamment à la lumière environnant permet aussi de contrôler la distance de travail entre un capteur optique et la surface corporelle. Une telle distance permet en outre de définir un point de référence pour la quantification des artefacts détectés et calculés par des algorithmes.

Ainsi, le dispositif électroportatif peut permettre la prise d'images et le transfert des données délivrées par son capteur optique à un système informatisé. Dans ce cas, l'unité de stockage peut être de type mémoire permanente pour conserver une information représentative de l'image et permettre son transfert ultérieur. L'unité de stockage peut comporter ainsi une capacité pour mémoriser un nombre conséquent d'images sans qu'il soit utile de transférer les données contenues dans l'unité de stockage. Une fois les photographies de la surface corporelle réalisées, les données peuvent donc être transférées vers un système informatisé afin de permettre leur analyse. Cette capacité de stockage peut également être utilisée pour conserver des informations telles que des codes d'identifications mais également des informations sur l'état du capteur optique, sur la calibration de l'organe d'éclairage ou encore sur d'éventuels paramètres physico-chimiques de la surface corporelle étudiée.

Par ailleurs, le système informatisé peut être associé à une base de données de traitements et produits traitants, permettant d'indiquer à l'utilisateur un traitement et/ou le produit le plus approprié à l'état de sa peau ou de ses cheveux, ce produit étant choisi parmi une gamme préenregistrée dans la base de données.

Selon une première variante, le système informatisé peut être l'ordinateur personnel de l'utilisateur du dispositif d'obturation. Dans ce cas, la prise d'image de la surface corporelle peut être réalisée à son domicile mais également dans un lieu public. La miniaturisation des composants électroniques fait que par système informatisé, on entend tout système aussi petit qu'il soit mais apte à stocker les données dans une mémoire de type permanente et à communiquer avec un serveur WEB. Un téléphone portable ou un organiseur portable peuvent ainsi être utilisés en tant que système informatisé.

Selon une seconde variante, le système informatisé peut être une borne installée à l'intérieur d'un magasin de produits cosmétiques. Le dispositif d'obturation peut notamment être celui du produit cosmétique contenu dans un flacon, tube ou analogue. Dans ce cas, l'utilisation du dispositif d'obturation peut se faire juste avant l'achat d'un produit. De cette manière, l'utilisateur peut connaître le produit le mieux adapté à l'état de sa peau ou de ses cheveux précisément au moment de l'achat du produit.

L'unité de transfert des données de l'image du dispositif électroportatif peut notamment utiliser des radiofréquences. Le protocole utilisé pour transmettre les données peut alors se présenter sous diverses variantes et notamment du type Bluetooth, Wifi ou Zigbee.

Un tel dispositif d'obturation n'est donc pas totalement autonome, cependant il comporte un logement adapté pour recevoir avec précision, et ce toujours avec le même positionnement par rapport à la surface corporelle, un dispositif électroportatif. Un tel dispositif électroportatif est avantageusement un Smartphone mais il peut aussi consister en un appareil photographique ou un lecteur multimédia tel une tablette tactile ou un lecteur MP3 ou MP4 notamment munis d'un capteur optique, d'une mémoire et d'un module de transfert de données sans fil.

Selon un premier mode de réalisation, le dispositif peut comporter :
- un organe d'éclairage apte à éclairer la surface corporelle lorsqu'elle est positionnée au niveau de la surface plane du corps, l'organe d'éclairage étant agencé dans une zone interne du volume étanche formé par le corps et le système optique ;
- un système de pilotage électronique de l'organe d'éclairage ;
- un organe électrique permettant de commander la mise sous tension électrique de l'organe d'éclairage.

L'organe d'éclairage permet quant à lui d'éclairer de façon sensiblement homogène la surface corporelle dont on souhaite prendre un cliché photographique. Si besoin, un traitement numérique des données issues du capteur optique permet également de corriger d'éventuels défauts d'homogénéité de l'organe d'éclairage. L'organe d'éclairage peut notamment comporter une source blanche ou monochromatique ou à bande spectrale étroite.

Par ailleurs, lorsque le dispositif n'est pas utilisé, l'organe électrique permet d'ouvrir un circuit de commande de l'organe d'éclairage pour ne pas consommer inutilement d'énergie électrique.

Selon un second mode de réalisation, la surface corporelle peut également être éclairée au moyen d'un organe d'éclairage équipant dispositif électroportatif. Son éclairage peut alors être direct ou bien diffus en passant par exemple au travers d'une cloison translucide ou transparente du corps dispositif d'obturation.

En outre, l'organe d'éclairage peut se présenter sous différentes formes. Ainsi, selon une première variante, l'organe d'éclairage peut comporter des diodes électroluminescentes dont le lobe principal d'émission est perpendiculaire par rapport à la surface plane du corps.

Ainsi, dans ce cas, les diodes électroluminescentes sont orientées de façon coaxiale par rapport à un axe optique du système optique permettant de grossir l'image. Les diodes sont alors réparties à la périphérie du système optique sur une zone du corps sensiblement parallèle à la surface plane sur laquelle la surface corporelle doit être placée pour prendre son image. Dans ce cas également, l'éclairage des diodes peut être direct ou bien diffus en passant par exemple au travers d'une cloison translucide ou transparente du corps dispositif d'obturation.

Selon une seconde variante, l'organe d'éclairage peut comporter des diodes électroluminescentes dont le lobe principal d'émission est parallèle par rapport à la surface plane du corps et en ce que le dispositif peut comporter un miroir semi-transparent permettant de réfléchir la lumière émise par les diodes électroluminescentes tout en laissant traverser la lumière émise par la surface corporelle.

Dans ce cas, les diodes sont agencées dans un logement situé en dessous du système optique et un miroir semi transparent ,qui peut également se présenter sous la forme d'une lame séparatrice inclinée à 45°, est agencé sur l'axe optique du système optique pour refléter la lumière émise par les diodes en direction de la surface corporelle.

Avantageusement, le dispositif peut comporter des moyens de mise au point de l'image de la surface corporelle.

En d'autres termes, il peut être envisagé de modifier le positionnement des éléments optiques constituant le système optique. Un système de glissière peut notamment être utilisé pour déplacer de façon indépendante ou combinée les lentilles formant le système optique afin d'ajuster la mise au point de l'image de la surface corporelle.

En pratique, le dispositif peut comporter un indicateur lumineux d'informations permettant à l'utilisateur de visualiser une mise sous tension du dispositif.

Autrement dit, lorsque le dispositif est sous tension, l'indicateur lumineux d'informations permet d'indiquer à l'utilisateur une information représentative de la mise sous tension et ainsi d'éviter une déperdition inutile de l'énergie électrique consommée par le dispositif d'obturation.

Dans d'autres variantes, des informations peuvent aussi être stockées le temps de leur affichage dans l'unité de stockage. Les moyens d'affichage peuvent dans ce cas être notamment à cristaux liquides Ils peuvent ainsi permettre d'afficher quantitativement un niveau de valeur fonction de l'image ou d'une mesure réalisée. D'autres types de moyens d'affichages peuvent également être utilisés et notamment des moyens utilisant des LED ou OLED.

Selon un mode de réalisation particulier, le dispositif peut comporter un cache amovible apte à être positionné au niveau de la surface plane du corps, le cache amovible comportant, au niveau d'une face interne destinée à être éclairée par l'organe d'éclairage, un étalon colorimétrique permettant d'effectuer une calibration d'un ensemble optique permettant de prendre l'image d'une surface corporelle. Un tel ensemble optique est constitué par le capteur optique, le système optique et le système d'éclairage. Il s'agit ainsi d'effectuer une calibration colorimétrique quel que soit le capteur optique utilisé et d'obtenir une uniformité d'éclairement.

Autrement dit, le cache amovible peut être positionné temporairement en regard du capteur optique pour permettre une correction colorimétrique de l'image générée et un étalonnage de l'ensemble optique. Un tel étalon colorimétrique permet ainsi une correction de l'uniformité de l'éclairement sur la surface corporelle qui est mémorisée dans l'unité de stockage du dispositif d'obturation ou dans le dispositif électroportatif équipé du capteur optique.

Un tel étalon colorimétrique peut être formé notamment par une mire imprimée sur une feuille de papier collée sur le cache amovible ou encore par le cache amovible lui-même lorsque celui-ci est teinté dans la masse.

L'étalon colorimétrique peut avantageusement comporter une échelle graduée permettant d'effectuer également une calibration spatiale, quel que soit le capteur optique utilisé pour prendre l'image de la surface corporelle.

Dans d'autres variantes, l'étalonnage colorimétrique peut également être obtenu directement au moyen d'une pompe formant mire de calibration du contenant. La forme, la position et la teinte de la pompe.

Selon un premier mode de réalisation, le dispositif peut comporter une source d'énergie autonome.

Dans ce premier cas, la source d'énergie autonome peut se présenter sous la forme d'une pile qui permet d'alimenter l'organe d'éclairage et le système de pilotage électronique de l'organe d'éclairage. Une telle pile peut également permettre d'alimenter le capteur optique et l'unité de stockage et de transfert des données lorsque ceux-ci sont intégrés au dispositif d'obturation.

Une telle source d'énergie électrique autonome peut également comporter une cellule photovoltaïque. Cette cellule permet d'alimenter l'organe d'éclairage et le système de pilotage. Dans ce cas, l'énergie est donc fournie par la lumière ambiante.

Dans une autre variante, la source d'énergie électrique autonome peut être un micro-générateur transformant une source d'énergie extérieure en énergie électrique par inductions, effet Hall, magnéto résistif, piézoélectrique, thermique ou mécanique.

Selon un second mode de réalisation, le dispositif peut comporter un connecteur électrique permettant une alimentation externe en énergie électrique du dispositif.

En d'autres termes, le dispositif d'obturation peut être connecté par liaison filaire à une source d'énergie extérieure qui peut être un dispositif électroportatif intégrant le capteur optique via un câble USB ou coaxial notamment.

Avantageusement, le dispositif peut comporter au moins un filtre apte à détecter un phénomène physique.

Un tel filtre peut notamment être agencé à proximité immédiate du capteur optique. Par ailleurs, ce filtre peut de se présenter sous différentes formes et être de diverses natures. Il peut également consister en un empilement de plusieurs filtres. De tels filtres permettent notamment de détecter des phénomènes physiques tels que :
- la réflexion ou l'absence de réflexion sur une surface au moyen d'une polarisation parallèle ou croisée avec des filtres polariseurs croisés ou parallèles ;
- l'absorption de la longueur d'onde émise comme par exemple avec de l'hémoglobine qui présente un pic d'absorption à 560 nm avec des filtres polariseurs croisés ou parallèles ;
- la fluorescence correspondant à un éclairement à une longueur d'onde donnée et une image capturée autour d'une autre longueur d'onde, dans ce cas plusieurs technologies de filtres peuvent être utilisées telles que notamment les filtres colorés du type gélatine ou verre teinté dans la masse, les filtres interférentiels ou holographiques et les lames dichroïques.

En pratique, le dispositif d'obturation peut comporter au moins un capteur sensible à une propriété physico-chimique de la surface corporelle de l'utilisateur.

Autrement dit, un capteur permet de fournir une information fonction d'une propriété physico-chimique de la surface corporelle. Le signal peut être reçu par une unité de traitement intégrée à l'intérieur du dispositif d'obturation, puis ce signal est traité de façon à permettre sa mémorisation dans l'unité de stockage interne ou externe au dispositif. L'unité de traitement et le capteur nécessitent d'être alimentés en énergie électrique pour délivrer un signal.

Dans un mode de réalisation particulier, le capteur sensible à une propriété physico-chimique peut être actif. Il délivre alors un signal représentatif d'un paramètre physico-chimique d'une surface corporelle seulement lorsque qu'il est alimenté en électricité.

Dans d'autres variantes, le capteur peut être passif.

Selon la mesure de la propriété physico-chimique à réaliser, le capteur peut-être choisi notamment parmi le groupe des capteurs :
- de pH ;
- d'empreinte cutanée, apte à mesurer la topographie de la surface cutanée à analyser ;
- d'humidité de la peau ;
- de température de la peau ;
- d'humidité de l'air ambiant ;
- de taux lipidique ;
- de déformation élastique de la surface cutanée à analyser.

Bien entendu, pour certaines applications, le dispositif d'obturation peut comporter plusieurs capteurs sensibles à une propriété physico-chimique choisis parmi ce groupe et aptes à mesurer des propriétés physico-chimiques différentes.

La mesure du pH de la peau permet de distinguer un pH élevé, de l'ordre de 5,5 et un pH plus acide, voisin de 5.

Le capteur d'humidité de la peau permet de mesurer de façon très précise le paramètre généralement qualifié de « perte insensible d'eau » ou en anglais « Trans Epidermal Water Loss », abrégé en TEWL.

Ce paramètre correspond à l'évaluation d'un phénomène indépendant de la transpiration, se traduisant par l'évaporation de l'eau depuis les couches sous-jacentes de l'épiderme. Cette mesure permet par exemple de contrôler le film hydrolipidique qui joue le rôle de fonction barrière de la peau, et de définir l'échelle de sécheresse cutanée de la peau.

Le capteur d'empreinte cutanée permet d'élaborer une mesure des différentes irrégularités de la surface de la peau. Cette mesure peut être réalisée selon différents principes tel qu'une mesure capacitive, piézorésistive, piézoélectrique, optique ou électromagnétique. La détermination de la topographie de la zone à analyser permet de mesurer la régularité de la peau, le nombre de rides, leur longueur, leur surface et leur profondeur moyenne. La surface globale des rides peut être déterminée en calculant la surface occupée par les rides moyennes et les rides profondes, correspondant respectivement aux rides dont la profondeur est comprise entre 150 et 200 micromètres, et supérieure à 200 micromètres.

Il est également possible de déterminer l'intensité des sillons principaux, de manière à rendre compte de la longueur des rides les plus profondes. La détermination du volume des rides principales permet de mesurer l'évolution dans le temps de ces rides.

La mesure de la rugosité de le peau est également un paramètre important, car il permet d'approcher globalement la notion de planéité de la peau en la caractérisant par une valeur d'amplitude moyenne qui est résultante des différents accidents de relief comparativement à une surface plane. La mesure de ce paramètre de rugosité, ainsi que de son évolution dans le temps, permet de mettre en évidence le lissage de la peau consécutif à un traitement particulier, qui peut être une crème ou une substance quelconque présente à l'intérieur du contenant.

La mesure de la température ou de l'humidité ambiante permet de corriger certaines mesures particulières, et notamment celle du taux d'humidité de la peau, c'est-à-dire la perte insensible d'eau. Elle permet également d'analyser un diagnostic par rapport aux conditions atmosphériques.

Le capteur de taux lipidique permet de déterminer le statut des lipides cutanés, notamment pour les peaux sèches. Cette mesure permet de distinguer les phénomènes de sécheresse cutanée, les phénomènes de production excessive de sébum.

Le capteur de déformation élastique de la surface corporelle à analyser permet de mesurer la fermeté et l'élasticité de la peau. Ce capteur de déformation fonctionne sur le principe de l'application d'une dépression sur une zone de peau, pendant une durée constante. Plusieurs aspirations successives peuvent être effectuées de manière à mesurer la profondeur de pénétration de la peau dans une partie de la carte. Plus précisément, cette mesure peut être effectuée par le biais de capteurs optiques ou à base de jauges de contraintes par exemple.

L'analyse des différentes mesures obtenues permet de distinguer les déformations instantanées, correspondant à un phénomène d'élasticité, ainsi que des déformations retardées, assimilables à un phénomène de viscosité.

Avantageusement le capteur est réalisé à partir de micro-systèmes. Une variante de capteurs actifs ou passifs peut fonctionner selon le principe des technologies de type MEMS , signifiant « Système Micro Electro Mécanique ». Ces capteurs sont donc réalisés selon des technologies utilisant des matériaux semi-conducteurs, isolants ou métalliques, et des méthodes d'usinage chimique employées dans le domaine de la micro-électronique. L'emploi de capteurs de type MEMS permet de concentrer un grand nombre de capteurs sur une zone particulièrement restreinte, implantée sur le dispositif d'obturation. Celui-ci permet d'obtenir des résultats représentatifs d'une zone localisée, homogène et caractéristique.

Selon la nature de la mesure à effectuer, les données relevées par le capteur peuvent, soit être affichées directement sur le dispositif d'obturation, soit être transférées vers un système informatisé, voire les deux.

Selon un mode de réalisation particulier, le dispositif peut comporter au moins un capteur sensible à une condition d'environnement du milieu extérieur.

Ainsi, un tel dispositif d'obturation peut permettre de mesurée des données telles que la température, le taux d'humidité ou le taux de rayonnement ultraviolet du milieu environnant. En effet, de tels paramètres extérieurs peuvent notamment influer sur l'image qui est prise par le capteur optique ou encore directement sur l'apparence visuelle de la surface corporelle.

Avantageusement, le dispositif peut comporter au moins un moyen de sélection permettant à l'utilisateur de sélectionner un mode de fonctionnement du dispositif.

Autrement dit, un tel moyen de sélection peut être utile lorsque le dispositif d'obturation est autonome et comporte un capteur optique ou d'autres capteurs notamment. Le moyen de sélection permet notamment de commander l'alimentation électrique de l'organe d'éclairage et le déclenchement de la prise d'image par le capteur optique.

En pratique, le dispositif peut comporter une pièce d'adaptation coopérant avec le corps, la pièce d'adaptation comportant le logement apte à recevoir le dispositif électroportatif et permettant de réaliser un positionnement précis d'un capteur optique du dispositif électroportatif spécifique par rapport au système optique du dispositif d'obturation.

Dans ce cas, le dispositif d'obturation peut comporter un corps formant un élément standard commercialisé en masse et permettant d'obturer l'ensemble des contenants d'une gamme de produit. La pièce d'adaptation est alors un accessoire destiné à rendre compatible un dispositif électroportatif spécifique avec le dispositif d'obturation standard. Une telle pièce d'adaptation peut alors être accouplée à un ou plusieurs dispositifs électroportatifs et permet de positionner de façon précise le capteur du dispositif électroportatif en regard du système optique du dispositif d'obturation.

Selon une première variante, la pièce d'adaptation peut comporter un orifice de forme complémentaire à la forme d'une section transversale du corps, le corps étant apte à être inséré dans l'orifice de la pièce d'adaptation.

Dans ce cas, la pièce d'adaptation est positionnée au niveau d'une extrémité du corps à proximité d'une surface parallèle à la surface plane sur laquelle la surface corporelle est destinée à être agencée. La pièce d'adaptation est alors insérée sur le corps du dispositif d'obturation par un mouvement de translation parallèle à l'axe optique du système optique.

Selon une seconde variante, la pièce d'adaptation peut être apte à être insérée dans le logement du dispositif, le logement étant agencé au niveau d'une face latérale du corps.

Le logement du corps est alors positionné au-dessus du système optique de façon à positionner le capteur optique en regard du système optique. La pièce d'adaptation est alors insérée dans le logement du corps du dispositif d'obturation par un mouvement de translation perpendiculaire à l'axe optique du système optique.

### Description sommaire des figures

La manière de réaliser l'invention ainsi que les avantages qui en découlent, ressortiront bien de la description du mode de réalisation qui suit, donné à titre indicatif et non limitatif, à l'appui des figures dans lesquelles :
- la figure 1 est une vue en perspective d'un flacon équipé d'un dispositif d'obturation conforme à l'invention ;
- les figures 2 à 6 sont des vues en coupe transversale de quatre variantes de dispositifs d'obturation conformes à l'invention.

### Manière de décrire l'invention

Comme déjà évoqué, l'invention concerne un dispositif d'obturation pour un contenant de façon à réaliser une image d'une surface corporelle qui peut être notamment une surface cutanée ou capillaire.

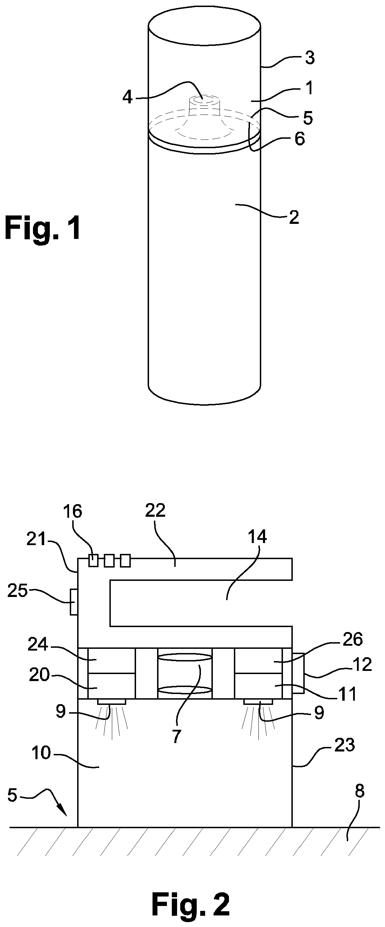

Tel que représentée à la figure 1, le dispositif d'obturation **1** permet de masquer et de protéger l'orifice **4** d'un contenant **2.** Un tel dispositif d'obturation **1** comporte un corps **3** muni d'une surface plane **5** destinée à coopérer avec une surface corporelle d'un utilisateur du dispositif **1.** Par ailleurs, lorsque que le dispositif d'obturation **1** est assemblé avec le contenant **2,** la surface plane **5** est agencée en regard d'une surface parallèle **6** du contenant **2** et permet de définir un volume sensiblement étanche au-dessus de l'orifice **4.**

Tel que représenté à la figure 2, le dispositif d'obturation **21** comporte un logement **14** agencé au-dessus d'un système optique **7.** Un tel logement **14** permet ainsi de positionner le capteur optique d'un dispositif électroportatif en regard du système optique **7.** Il permet ainsi de prendre une image d'une surface corporelle **8** positionnée au contact de la surface plane **5.**

Dans la variante représentée, un organe d'éclairage **9** peut être intégré au dispositif d'obturation **21** et de façon à éclairer la surface corporelle lors de la prise de l'image. Dans cette variante l'organe d'éclairage **9** est constitué de diodes électroluminescentes agencées à la périphérie du système optique 7 et en étant orientées de façon à ce que leur lobe principal d'émission est perpendiculaire par rapport à la surface plane **5** du corps **23.**

Un tel dispositif d'obturation **21** comporte également un système de pilotage électronique **11** de l'organe d'éclairage **9,** un organe électrique **12** permettant de commander la mise sous tension électrique de l'organe d'éclairage **9.** Dans cette variante totalement autonome du dispositif d'obturation **21,** une unité de stockage **24** permettant de mémoriser l'image, une unité de transfert **26** des données de l'image et une source d'énergie autonome **20** sont également intégrés dans le corps **23.**

Un tel dispositif d'obturation **21** comporte également un indicateur lumineux d'informations **16** permettant à l'utilisateur de visualiser une mise sous tension du dispositif **21** et notamment de l'organe d'éclairage **9.** Un moyen de sélection **25** permet quant à lui à l'utilisateur de sélectionner le mode de fonctionnement du dispositif **21** et notamment de le type d'image qu'il souhaite effectuer en fonction du type de surface corporelle ou encore en fonction du type de produit contenu dans un flacon ou tube.

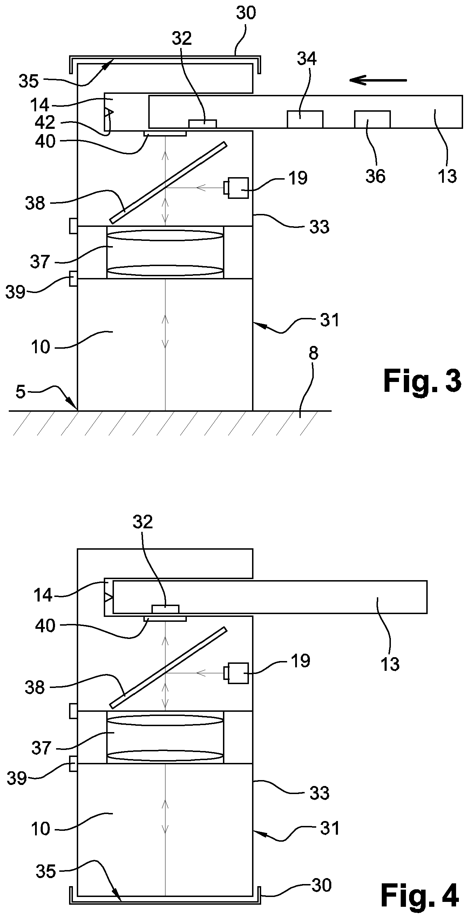

Tel que représenté à la figure 3, le corps **33** d'un dispositif d'obturation **31** comporte un logement **14** dans lequel un dispositif électroportatif **13** peut être inséré de façon à positionner un capteur optique **32** en regard d'un système optique **37.** Un tel dispositif électroportatif **13** comporte également une unité de stockage **34** pour mémoriser l'image et une unité de transfert **36** des données de l'image.

L'introduction du dispositif électroportatif **13** dans le fond du logement **14** permet d'actionner un organe électrique **42** permettant de commander la mise sous tension électrique d'un organe d'éclairage **19.** Un tel organe électrique peut se présenter sous la forme d'un contacteur tel un interrupteur.

Dans cette variante, l'organe d'éclairage **19** se présente sous la forme d'une diode électroluminescente orientée de façon à ce que le lobe principal d'émission soit parallèle par rapport à la surface plane **5** du corps **33.** Par ailleurs, le dispositif **31** comporte également un miroir semi-transparent **38** permettant de réfléchir la lumière émise par la diode électroluminescente tout en laissant traverser la lumière émise par la surface corporelle **8.** Un tel miroir semi-transparent **38** est incliné à 45° par rapport à l'axe optique du système optique **37.**

De plus, le dispositif d'obturation **31** comporte des moyens de mise au point **39** de l'image de la surface corporelle **8.** De tels moyens de mise au point **39** peuvent notamment comporter des glissières permettant de déplacer en translation des lentilles formant le système optique le long de l'axe optique.

Par ailleurs, le dispositif d'obturation **31** comporte également un cache amovible **30** agencé au niveau d'une surface supérieure du dispositif d'obturation **31.** Un tel cache amovible **30** comporte, au niveau d'une face interne **35** un étalon colorimétrique permettant d'effectuer une calibration d'un capteur optique **32** permettant de prendre l'image d'une surface corporelle **8.**

Tel que représenté à la figure 4, la face interne **35** du cache amovible **30** est destinée à être éclairée par l'organe d'éclairage **19.** Pour ce faire, on positionne le cache amovible **30** au niveau de la surface plane **5** du dispositif d'obturation **31.**

Par ailleurs, le dispositif d'obturation **31** comporte également un filtre polarisant **40** permettant de supprimer des reflets de la lumière émise par l'organe d'éclairage **19** sur la surface corporelle **8.**

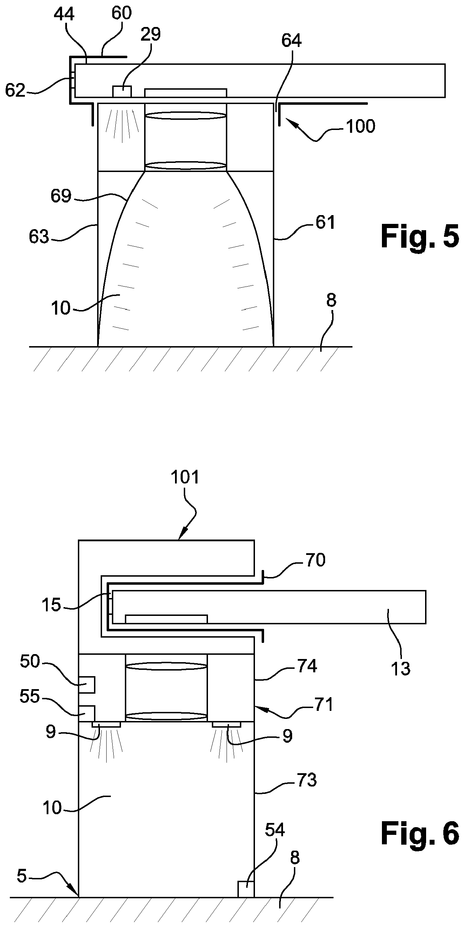

Tels que représentés aux figures 5 et 6, le dispositif d'obturation conforme à l'invention peut également comporter une pièce d'adaptation permettant de réaliser un positionnement précis d'un capteur optique d'un dispositif électroportatif spécifique par rapport au système optique du dispositif d'obturation.

Ainsi, selon une première variante, telle que représentée à la figure 5, la pièce d'adaptation **60** peut comporter un orifice **64** dont la forme peut être prismatique ou circulaire de façon à être complémentaire avec la forme de la section transversale du corps **63.**

Dans ce cas, un organe électrique **62,** qui permet de commander la prise de photo et/ou la mise sous tension électrique de l'organe d'éclairage **29,** est agencé en fond d'une gorge formant logement pour le dispositif électroportatif équipé du capteur optique.

Dans ce cas, la lumière est donc émise par un organe d'éclairage solidaire du dispositif électroportatif **13.** La lumière ainsi émise peut alors se diffuser au travers d'une surface **69** translucide du corps **63** de façon à éclairer la surface corporelle **8** avec une bonne homogénéité.

Selon une autre variante, telle que représentée à la figure 6, le dispositif d'obturation peut comporter une pièce d'adaptation **70** insérée dans un logement **15** dont la forme est sensiblement prismatique. Un tel logement **15** est avantageusement orienté de façon parallèle à la surface plane **5** du corps **73.**

Par ailleurs, un tel dispositif d'obturation **71** comporte un connecteur électrique **50** permettant une alimentation externe en énergie électrique du dispositif **71.** Un tel connecteur électrique **50** permet notamment la connexion d'un câble de type usb ou coaxial.

Un tel dispositif d'obturation **71** comporte également un capteur **54** sensible à une propriété physico-chimique de la surface corporelle de l'utilisateur et un capteur **55** sensible à une condition d'environnement du milieu extérieur.

Par ailleurs, selon un premier mode de réalisation, les données des images peuvent alors être analysées directement par le dispositif d'obturation ou le dispositif électroportatif pour réaliser une analyse et notamment comparer l'image avec des images prises antérieurement et stockées dans des bases de données afin de prescrire un produit adapté à la surface corporelle.

Selon un second mode de réalisation, le dispositif d'obturation ou le dispositif électroportatif peut servir de passerelle vers un serveur central apte à récupérer les données issues de chaque utilisateur. Une base de données d'images est alors implémentée par les nouvelles images prises par chacun des utilisateurs.

Par conséquent, il est possible à l'utilisateur muni d'un dispositif d'obturation, accessoirement d'un dispositif électroportatif, et d'une connexion Internet, de connaître à tout instant et n'importe où, l'historique de l'état de sa surface corporelle et de visualiser cet historique par des courbes notamment.

En fonction des données reçues par le serveur, l'emploi d'un produit ou d'un traitement adapté peut être proposé à l'utilisateur. Il peut alors choisir de commander directement le produit en question et le recevoir à son domicile sans avoir besoin de se rendre dans un lieu de vente.

Par exemple, et dans un premier temps, la détermination du produit cosmétique le plus adapté aux propriétés physico-chimiques de la peau de l'utilisateur, se fait par confrontation de ces résultats avec des critères de diagnostics élaborés notamment par une équipe scientifique. Ces critères de diagnostics font eux même partie d'une base de données de diagnostics, qui peut être mise à jour et enrichie par l'analyse statistique de mesures antérieures mais également par les analyses de dermatologues équipés d'appareils d'analyse (par exemple selon le brevet WO 03/037184) connectés au serveur central.

Il ressort de ce qui précède qu'un dispositif d'obturation et l'ensemble conformes à l'invention présentent de multiples avantages, notamment :
- L'image de la surface corporelle peut permettre de diagnostiquer un éventuel symptôme, d'évaluer et de quantifier la variation de ce symptôme dans le temps, d'indiquer une marche à suivre quant à l'utilisation du produit et,le cas échéant, d'indiquer quels produits de traitement et/ou traitements complémentaires seraient les plus appropriés pour modifier l'aspect visuel de la surface corporelle et/ou traiter le symptôme diagnostiqué
- il permet, à lui seul, de réaliser un traitement à base de chromatothérapie par exemple. En effet, des diodes électroluminescentes multi-spectrales peuvent permettre d'effectuer une analyse multi-spectrale de la surface corporelle. Le dispositif peut ensuite permettre d'activer une substance active, d'accélérer sa pénétration dans la surface corporelle et d'accélérer son action.
- ils permettent de rendre financièrement accessible, à un grand nombre de personnes, l'analyse d'une image d'une surface corporelle ;
- ils sont intégrés au packaging du contenant de la substance permettant de traiter ou plus simplement de modifier l'aspect esthétique de la surface corporelle ;
- ils permettent d'acheter dans un magasin ou d'appliquer un produit cosmétique, en fonction du besoin réel de la peau à l'instant où l'image de la surface corporelle est effectuée.

## Revendications

1. Dispositif d'obturation (1, 21, 31, 61 71) d'un contenant (2), ledit dispositif (1, 21, 31, 61, 71) comportant un corps (3, 23, 33, 63, 73) apte à recouvrir et protéger un orifice (4) dudit contenant (2), ledit corps (3, 23, 33, 63, 73) présentant une surface plane (5) destinée à venir au contact d'une surface plane (6) parallèle dudit contenant (2), ledit dispositif d'obturation (1, 21, 31, 61, 71) comporte :
- un système optique (7, 37) permettant à la fois de définir un volume étanche au-dessus dudit orifice (4) du contenant (2) et de réaliser une image grossie d'une surface corporelle (8) d'un utilisateur dudit dispositif (1, 21, 31, 61, 71), lorsque ladite surface corporelle (8) est positionnée au contact de ladite surface plane (5) dudit corps (3, 23, 33, 63, 73) ; le dispositif d'obturation est **caractérisé en ce qu'**il comporte:
- un logement (14, 15, 44) apte à recevoir un dispositif électroportatif (13) comportant :
• un capteur optique (32) permettant de prendre ladite image de ladite surface corporelle (8), ledit logement (14, 15) étant agencé de telle sorte que le capteur optique (32) du dispositif électroportatif (13) est en regard du système optique (7, 37) ;
• une unité de stockage (34) permettant de mémoriser ladite image, et ;
• une unité de transfert des données (36) de ladite image.

2. Dispositif selon la revendication 1, **caractérisé en ce qu'**il comporte :
- un organe d'éclairage (9, 19) apte à éclairer ladite surface corporelle (8) lorsqu'elle est positionnée au niveau de la surface plane (5) dudit corps (3, 23, 33, 53, 63, 73), ledit organe d'éclairage (9, 19) étant agencé dans une zone interne (10) dudit volume étanche formé par ledit corps (3, 23, 33, 53, 63, 73) et ledit système optique (7) ;
- un système de pilotage électronique (11) dudit organe d'éclairage (9, 19) ;
- un organe électrique (12, 42, 62) permettant de commander la mise sous tension électrique dudit organe d'éclairage (9, 19).

3. Dispositif selon la revendication 1, **caractérisé en ce qu'**il comporte un capteur optique (22) permettant de prendre ladite image de ladite surface corporelle (8), une unité de stockage (24) permettant de mémoriser ladite image et une unité de transfert (26) des données de ladite image.

4. Dispositif selon la revendication 1, **caractérisé en ce que** ledit organe d'éclairage (9) comporte des diodes électroluminescentes dont le lobe principal d'émission est perpendiculaire par rapport à ladite surface plane(5) dudit corps (23, 73).

5. Dispositif selon la revendication 1, **caractérisé en ce que** ledit organe d'éclairage (19) comporte des diodes électroluminescentes dont le lobe principal d'émission est parallèle par rapport à ladite surface plane (5) dudit corps (33, 53) et **en ce que** ledit dispositif (31, 51) comporte un miroir semi-transparent (38) permettant de réfléchir la lumière émise par lesdites diodes électroluminescentes tout en laissant traverser la lumière émise par ladite surface corporelle (8).

6. Dispositif selon la revendication 1, **caractérisé en ce qu'**il comporte des moyens de mise au point (39) de ladite image de ladite surface corporelle (8).

7. Dispositif selon la revendication 1, **caractérisé en ce qu'**il comporte un indicateur lumineux d'informations (16) permettant à l'utilisateur de visualiser une mise sous tension dudit dispositif (21).

8. Dispositif selon la revendication 1, **caractérisé en ce qu'**il comporte un cache amovible (30) apte à être positionné au niveau de ladite surface plane (5) dudit corps (33), ledit cache amovible (30) comportant, au niveau d'une face interne (35) destinée à être éclairée par l'organe d'éclairage (19), un étalon colorimétrique permettant d'effectuer une calibration d'un ensemble optique comportant notamment ledit capteur optique (32), ledit système optique (37), et ledit système d'éclairage (19)..

9. Dispositif selon la revendication 1, **caractérisé en ce qu'**il comporte une source d'énergie autonome (20).

10. Dispositif selon la revendication 1, **caractérisé en ce qu'**il comporte un connecteur électrique (50) permettant une alimentation externe en énergie électrique dudit dispositif (51).

11. Dispositif selon la revendication 1, **caractérisé en ce qu'**il comporte au moins un filtre (40) apte à détecter un phénomène physique.

12. Dispositif selon la revendication 1, **caractérisé en ce qu'**il comporte au moins un capteur (54) sensible à une propriété physico-chimique de ladite surface corporelle (8) de l'utilisateur.

13. Dispositif selon la revendication 1, **caractérisé en ce qu'**il comporte au moins un capteur (55) sensible à une condition d'environnement du milieu extérieur.

14. Dispositif selon la revendication 1, **caractérisé en ce qu'**il comporte au moins un moyen de sélection (25) permettant à l'utilisateur de sélectionner un mode de fonctionnement du dispositif (21).

15. Dispositif selon la revendication 1, **caractérisé en ce qu'**il comporte une pièce d'adaptation (60, 70) coopérant avec ledit corps (63, 73), ladite pièce d'adaptation (60, 70) comportant ledit logement (14, 15, 44) apte à recevoir un dispositif électroportatif (13) et permettant de réaliser un positionnement précis d'un capteur optique (32) d'un dispositif électroportatif (13) spécifique par rapport audit système optique (7, 37) dudit dispositif d'obturation (1, 61, 71).

16. Dispositif selon la revendication 15, **caractérisé en ce que** la pièce d'adaptation (60) comporte un orifice (64) de forme complémentaire à la forme d'une section transversale dudit cops (63), ledit corps (63) étant apte à être inséré dans l'orifice (64) de ladite pièce d'adaptation (60).

17. Dispositif selon la revendication 15, **caractérisé en ce que** la pièce d'adaptation (70) est apte à être insérée dans ledit logement (14, 15) dudit dispositif (21, 71), ledit logement (14, 15) étant agencé au niveau d'une face latérale (74) dudit corps (73).

## Patentansprüche

1. Vorrichtung (31) zum Abdichten (1, 21, 31, 61, 71) eines Behälters (2), wobei diese Vorrichtung (1, 21, 31, 61, 71) ein Gehäuse (3, 23, 33, 63, 73) aufweist, das eine Öffnung (4) dieses Behälters (2) abdecken und schützen kann, wobei das Gehäuse (3, 23, 33, 63, 73) eine ebene Oberfläche (5) aufweist, die in Kontakt mit einer zu diesem Behälter (2) parallelen ebenen Oberfläche (6) kommen soll, diese Abdichtungsvorrichtung (1, 21, 31, 61, 71):
- ein optisches System (7, 37) umfasst, das es ermöglicht, sowohl ein dichtes Volumen über der Öffnung (4) des Behälters (2) zu definieren, als auch ein vergrößertes Bild einer Körperoberfläche (8) eines Benutzers der Vorrichtung (1, 21, 31, 61, 71) zu erzeugen, wenn die Körperoberfläche (8) in Kontakt mit der ebenen Oberfläche (5) dieses Gehäuses (3, 23, 33, 63, 73) positioniert ist;
wobei diese Abdichtungsvorrichtung **dadurch gekennzeichnet ist, dass** sie:
- einen Halter (14, 15, 44) enthält, der eine tragbare elektrische Vorrichtung (13) aufnehmen kann, die:
- einen optischen Sensor (32), mit dem dieses Bild der Körperoberfläche (8) aufgenommen werden kann, wobei dieser Halter (14, 15) in einer Art und Weise angeordnet ist, dass der optische Sensor (32) der tragbaren elektrischen Vorrichtung (13) sich gegenüber dem optischen System (7, 37) befindet;
- eine Speichereinheit (34), mit der dieses Bild gespeichert werden kann, und;
- eine Einheit zur Übertragung der Daten (36) dieses Bildes umfasst.

2. Vorrichtung gemäß Anspruch 1, **dadurch gekennzeichnet, dass** sie enthält:
- ein Beleuchtungselement (9, 19), das in der Lage ist, diese Körperoberfläche (8) zu beleuchten, wenn sie in Höhe der ebenen Fläche (5) dieses Gehäuses (3, 23, 33, 53, 63, 73) positioniert ist, wobei dieses Beleuchtungselement (9, 19) in einer Innenzone (10) dieses dichten Raums angeordnet ist, das von diesem Körper (3, 23, 33, 53, 63,73) und diesem optischen System (7) gebildet wird;
- ein elektronisches Steuerungssystem (11) für dieses Beleuchtungselement (9, 19);
- ein elektrisches Element (12, 42, 62), mit dem das Einschalten der elektrischen Spannung dieses Beleuchtungselementes (9, 19) gesteuert werden kann.

3. Vorrichtung gemäß Anspruch 1, **dadurch gekennzeichnet, dass** sie einen optischen Sensor (22), mit der dieses Bild dieser Körperoberfläche (8) aufgenommen werden kann, wobei eine Speichereinheit (24), mit der dieses Bild gespeichert werden kann, und eine Einheit zur Übertragung der Daten (26) dieses Bildes enthält.

4. Vorrichtung nach Anspruch 1, **dadurch gekennzeichnet, dass** dieses Beleuchtungselement (9) Leuchtdioden enthält, deren Hauptrichtwirkung senkrecht zu dieser ebenen Fläche (5) dieses Körpers (23, 73) liegt.

5. Vorrichtung nach Anspruch 1, **dadurch gekennzeichnet, dass** dieses Beleuchtungselement (19) Leuchtdioden enthält, deren Hauptrichtwirkung parallel zu dieser ebenen Fläche (5) dieses Gehäuses (33, 53) liegt und dass diese Vorrichtung (31, 51) einen halbdurchlässigen Spiegel (38) enthält, mit dem das von diesen Leuchtdioden ausgesandte Licht reflektiert werden kann, während gleichzeitig das von dieser Körperfläche (8) emittierte Licht durchgelassen wird.

6. Vorrichtung gemäß Anspruch 1, **dadurch gekennzeichnet, dass** sie Mittel zur Bearbeitung (39) dieses Bilds der Körperfläche (8) enthält.

7. Vorrichtung gemäß Anspruch 1, **dadurch gekennzeichnet, dass** sie eine Informations-Leuchtanzeige (16) enthält, mit der der Nutzer das Einschalten der Spannung dieser Vorrichtung (21) erkennen kann.

8. Vorrichtung gemäß Anspruch 1, **dadurch gekennzeichnet, dass** sie eine abnehmbare Abdeckung (30) enthält, die in Höhe dieser ebenen Fläche (5) dieses Gehäuses (33) positioniert werden kann, wobei diese abnehmbare Abdeckung (30), in Höhe einer Innenseite (35), die vom Beleuchtungselement (19) beleuchtet werden soll, eine Farbvergleichstafel enthält, mit der eine Kalibrierung einer optischen Baugruppe vorgenommen werden kann, die insbesondere diesen optischen Sensor (32), dieses optische System (37) und dieses Beleuchtungssystem (19) enthält.

9. Vorrichtung gemäß Anspruch 1, **dadurch gekennzeichnet, dass** sie eine autonome Energiequelle (20) enthält.

10. Vorrichtung gemäß Anspruch 1, **dadurch gekennzeichnet, dass** sie eine elektrische Verbindung (50) enthält, über die diese Vorrichtung (51) extern mit elektrischer Energie versorgt werden kann.

11. Vorrichtung gemäß Anspruch 1, **dadurch gekennzeichnet, dass** sie mindestens einen Filter (40) enthält, mit dem ein physisches Phänomen erkannt werden kann.

12. Vorrichtung gemäß Anspruch 1, **dadurch gekennzeichnet, dass** sie mindestens einen Sensor (54) enthält, der gegenüber einer physikalisch-chemischen Eigenschaft dieser Körperfläche (8) des Nutzers empfindlich ist.

13. Vorrichtung gemäß Anspruch 1, **dadurch gekennzeichnet, dass** sie mindestens einen Sensor (55) enthält, mit dem eine Umweltbedingung außen erkannt werden kann.

14. Vorrichtung gemäß Anspruch 1, **dadurch gekennzeichnet, dass** sie mindestens eine Auswahlvorrichtung (25) enthält, mit der der Nutzer einen Funktionsmodus der Vorrichtung (21) auswählen kann.

15. Vorrichtung gemäß Anspruch 1, **dadurch gekennzeichnet, dass** sie einen Adapter (60, 70) enthält, der mit diesem Gehäuse (63, 73) zusammenarbeitet, wobei dieser Adapter (60, 70) den genannten Halter (14, 15, 44), der eine tragbare elektrische Vorrichtung (13) aufnehmen kann, enthält und eine genaue Positionierung eines optischen Sensors (32) einer tragbaren elektrischen Vorrichtung (13) erlaubt, die für dieses optische System (7, 37) dieser Abdichtungsvorrichtung (1 ,61, 71) spezifisch ist.

16. Vorrichtung gemäß Anspruch 15, **dadurch gekennzeichnet, dass** der Adapter (60) eine Öffnung (64) enthält, die zur Form eines Querschnitts dieses Körpers (63) komplementär ist, wobei dieser Körper (63) in die Öffnung (64) dieses Adapters (60) eingeführt werden kann.

17. Vorrichtung gemäß Anspruch 15, **dadurch gekennzeichnet, dass** der Adapter (70) in den Halter (14, 15) dieser Vorrichtung (21, 71) eingeführt werden kann, dieser Halter (14, 15) in Höhe einer Seitenfläche (74) dieses Gehäuses (73) angeordnet ist.

## Claims

1. Device for sealing (1, 21, 31, 61, 71) a container (2), the which device (1, 21, 31, 61, 71) incorporates a body (3, 23, 33, 63, 73) able to cover and protect an aperture (4) in the said container (2), the which body (3, 23, 33, 63, 73) has a flat surface (5) intended to come into contact with a parallel flat surface (6) on the container (2), with the said sealing device (1, 21, 31, 61, 71) incorporating:
• an optical system (7, 37) allowing the definition of a sealed volume above the said aperture (4) in the container (2) and the production of an enlarged image of a body surface (8) of a user of the said device (1, 21, 31, 61, 71) when the said body surface (8) is positioned in contact with the said flat surface (5) of the said body (3, 23, 33, 63, 73).
The sealing device is **characterized in that** it incorporates:
• a housing (14, 15, 44) able to accommodate a portable electric device (13) incorporating:
• an optical sensor (32) allowing the taking of the said image of the said body surface (8), with the said housing (14, 15) being designed such that the optical sensor (32) of the portable electric device (13) is opposite the optical system (7, 37);
• a storage unit (34) allowing memory storage of the said image; and
• a unit for transferring the data (36) of the said image.

2. Device in accordance with claim 1, **characterized by** the fact that it incorporates:
• a lighting component (9, 19) able to illuminate the said body surface (8) when it is positioned level with the flat surface (5) of the said body (3, 23, 33, 53, 63, 73), with the said lighting component (9, 19) being incorporated within an interior area (10) of the said sealed volume formed by the said body (3, 23, 33, 53, 63, 73) and the said optical system (7);
• an electronic system (11) for steering the said lighting component (9, 19);
• an electrical component (12, 42, 62) allowing one to command the electrical powering-on of the said lighting component (9, 19).

3. Device in accordance with claim 1, **characterized by** the fact that it incorporates an optical sensor (22) allowing the taking of the said image of the said body surface (8), a storage unit (24) allowing memory storage of the said image, and a transfer unit (26) for transferring the data of the said image.

4. Device in accordance with claim 1, **characterized by** the fact that the said lighting component (9) incorporates light-emitting diodes of which the principal emission lobe is perpendicular to the said flat surface (5) of the said body (23, 73).

5. Device in accordance with claim 1, **characterized by** the fact that the said lighting component (19) incorporates light-emitting diodes of which the principal emission lobe is parallel with the said flat surface (5) of the said body (33, 53), and by the fact that the said device (31, 51) incorporates a semi-transparent mirror (38) that reflects the light emitted by the said light-emitting diodes while allowing the light emitted by the said body surface (8) to pass through.

6. Device in accordance with claim 1, **characterized by** the fact that it incorporates a focusing system (39) for focusing the said image of the said body surface (8).

7. Device in accordance with claim 1, **characterized by** the fact that it incorporates an illuminating information indicator (16) allowing the user to view a powering-on of the said device (21).

8. Device in accordance with claim 1, **characterized by** the fact that it incorporates a removable cache (30) that can be positioned level with the said flat surface (5) of the said body (33), the which said removable cache (30) incorporates - on an interior face (35) designed to be illuminated by the lighting component (19) - a colorimetric calibration system allowing one to perform a calibration of an optical system, notably incorporating the said optical sensor (32), the said optical system (37), and the said lighting system (19).

9. Device in accordance with claim 1, **characterized by** the fact that it incorporates an autonomous power source (20).

10. Device in accordance with claim 1, **characterized by** the fact that it incorporates an electrical connector (50) providing an external electrical power source for the said device (51).

11. Device in accordance with claim 1, **characterized by** the fact that it incorporates at least one filter (40) able to detect a physical phenomenon.

12. Device in accordance with claim 1, **characterized by** the fact that it incorporates at least one sensor (54) sensitive to a physical/chemical property of the said body surface (8).

13. Device in accordance with claim 1, **characterized by** the fact that it incorporates at least one sensor (55) sensitive to an environmental condition of the exterior environment.

14. Device in accordance with claim 1, **characterized by** the fact that it incorporates at least one selection system (25) allowing the user to select a mode of operation of the device (21).

15. Device in accordance with claim 1, **characterized by** the fact that it incorporates an adapter part (60, 70) interacting with the said body (63, 73), the which said adapter part (60, 70) incorporates the said housing (14, 15, 44) able to accommodate a portable electric device (13) and allowing one to perform a precise positioning of an optical sensor (32) of a portable electric device (13) specific to the said optical system (7, 37) of the said sealing device (1, 61, 71).

16. Device in accordance with claim 15, **characterized by** the fact that the adapter part (60) incorporates an aperture (64) of a shape complementing the shape of a transversal section of the said body (63), the which body (63) is able to be inserted in the said aperture (64) of the said adapter part (60).

17. Device in accordance with claim 15, **characterized by** the fact that the adapter part (70) can be inserted in the said housing (14, 15) of the said device (21, 71), the which housing (14, 15) is incorporated in one lateral face (74) of the said body (73).
